# EUROPÄISCHE PATENTSCHRIFT

(11) **EP 0 932 403 B1**
(45) Veröffentlichungstag und Bekanntmachung des Hinweises auf die Patenterteilung: **30.11.2005**
(21) Anmeldenummer: 97913132.3
(22) Anmeldetag: 01.10.1997
(51) Int. Cl.: A61K 31/415, C07D 405/04, C07D 409/04, A61P 9/00, A61P 9/10, A61P 25/00, A61P 43/00

(54) **1-BENZYL-3-(SUBSTITUIERTES-HETARYL)-KONDENSIERTEN PYRAZOL-DERIVATEN ZUR BEHANDLUNG VON ERKRANKUNGEN DES HERZ-KREISLAUFSYSTEMS UND DES ZENTRALNERVENSYSTEMS**
CONDENSED (HETARYL-SUBSTITUTED) 1-BENZYL-3-PYRAZOL DERIVATES FOR TREATING DISEASES OF THE CARDIOVASCULAR AND CENTRAL NERVOUS SYSTEMS
UTILISATION DE 1-BENZYL-3-DERIVES CONDENSES (HETARYL-SUBSTITUES) DE PYRAZOL POUR LE TRAITEMENT DES AFFECTIONS DU SYSTEME CARDIOVASCULAIRE ET DU SYSTEME NERVEUX CENTRAL

(30) Priorität: 14.10.1996 DE 19642255
(43) Veröffentlichungstag der Anmeldung: 04.08.1999
(73) Patentinhaber: Bayer HealthCare AG, 51368 Leverkusen (DE)
(72) Erfinder: FÜRSTNER, Chantal, D-45470 Mülheim (DE); STRAUB, Alexander, D-42113 Wuppertal (DE); NIEWÖHNER, Ulrich, D-42929 Wermelskirchen (DE); JAETSCH, Thomas, D-50668 Köln (DE); FEURER, Achim, D-51519 Odenthal (DE); KAST, Raimund, D-42389 Wuppertal (DE); STASCH, Johannes-Peter, D-42651 Solingen (DE); PERZBORN, Elisabeth, D-42327 Wuppertal (DE); HÜTTER, Joachim, D-42349 Wuppertal (DE); DEMBOWSKY, Klaus, D-42115 Wuppertal (DE)
(86) Internationale Anmeldenummer: PCT/EP1997/005381
(87) Internationale Veröffentlichungsnummer: WO 1998/016223

(56) Entgegenhaltungen:
- EP-A- 0 470 039
- EP-A- 0 667 345
- S.-M. YU ET AL.: "Inhibition of Platelet Function by A02131-1, a Novel Inhibitor of cGMP-Specific Phosphodiesterase, In Vitro and In Vivo" BLOOD, Bd. 87, Nr. 9, 1.Mai 1996, Seiten 3758-3767, XP002056082 in der Anmeldung erwähnt
- C.-C. WU ET AL.: "YC-1 inhibited human platelet aggregation through NO-independent activation of soluble guanylate cyclase" BRITISH JOURNAL OF PHARMACOLOGY , Bd. 116, Nr. 3, 1995, Seiten 1973-1978, XP002056083 in der Anmeldung erwähnt

## Beschreibung

Die vorliegende Erfindung betrifft die neue Verwendung von teilweise bekannten 1-Benzyl-3-(substituierten-hetaryl)-kondensierten Pyrazol-Derivaten als Arzneimittel, neue Wirkstoffe, insbesondere ihre Verwendung als Vasodilatoren, gegebenenfalls in Kombination mit organischen Nitraten und NO-Donoren und gegebenenfalls in Kombination mit Verbindungen, die den Abbau von cGMP inhibieren.

Es ist bereits bekannt, daß 1-Benzyl-3-(substituierte hetaryl)-kondensierte Pyrazol-Derivate die stimulierte Thrombozytenaggregadon in vitro inhibieren (vgl. EP-667 345 Al; C.-C. Wu et al., Br. J. Pharmacol. 1995; 116: 1973 - 1978; F.-N. Ko et al., Blood 1994; 84: 4226 - 4233; S.-U. Yu et al., Blood 1996, 87: 3758 - 3767).

Es wurde nun überraschenderweise gefunden, daß 1-Benzyl-3-(substituierte hetaryl)-kondensierte Pyrazol-Derivate der allgemeinen Formel (I), 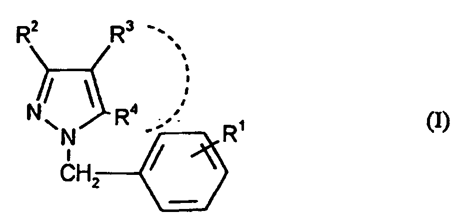 in welcher
- R¹: für Wasserstoff, Halogen, Hydroxy oder C₁-C₃-Alkyl oder C₁-C₃-Alkoxy steht,
- R²: für einen Rest der Formel 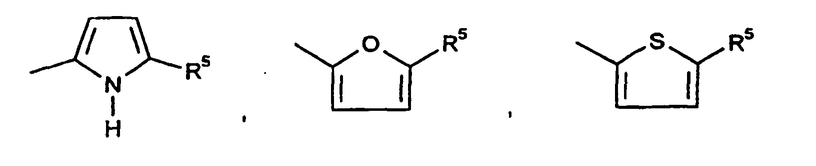 oder 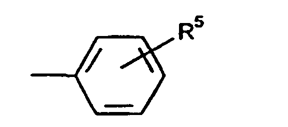 steht,
worin
R⁵ Wasserstoff, Halogen, Carboxyl, C₁-C₃-Alkyl, C₁-C₃-Alkoxycarbonyl oder einen Rest der Formel -CH₂-OR⁶ bedeutet,
worin
R⁶ Wasserstoff oder C₁-C₃-Alkyl bedeutet,
- R³ und R⁴: gemeinsam einen Rest der Formel 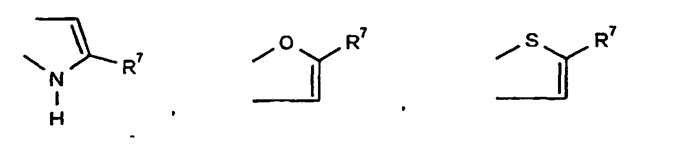 oder 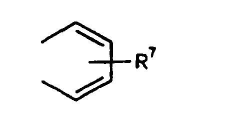 bilden,
worin
R⁷ Wasserstoff, Halogen, Hydroxy, C₁-C₃-Alkyl oder C₁-C₃-Alkoxy bedeutet,
und deren isomere Formen und Salze,
neben ihren schwachen antiaggregatorischen Eigenschaften eine ausgeprägte vasodilatorische Wirkung, insbesondere eine Blutdrucksenkung zeigen. Sie sind somit geeignet zur Behandlung von speziellen Erkrankungen des Herz-KreislaufSystems, insbesondere zur Behandlung verschiedener Formen der Angina pectoris, des Myokardinfarktes, der Herzinsuffizienz, der Arteriosklerose, Stroke und der Hypertonie.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall-oder Ammoniumsalze der erfindungsgemäßen Verbindungen sein, wenn sie eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.
- C₁-C₃-Alkyl: steht im Rahmen der Erfindung für einen geradkettigen oder verzweigten Kohlenwasserstoffrest mit 1 bis 3 Kohlenstoffatomen. Beispielsweise seien genannt: Methyl, Ethyl, Propyl und Isopropyl.
- C₁-C₃-Alkoxy: steht im Rahmen der Erfindung für einen geradkettigen oder verzweigten Alkoxyrest mit 1 bis 3 Kohlenstoffatomen. Beispielsweise seien genannt: Methoxy, Ethoxy, Propoxy und Isopropoxy.
- C₁-C₃-Alkoxycarbonyl: steht im Rahmen der Erfindung für einen geradkettigen oder verzweigten Alkoxycarbonylrest mit 1 bis 3 Kohlenstoffatomen. Beispielsweise seien genannt: Methoxycarbonyl, Ethoxycarbonyl, Propoxycarbonyl und Isopropoxycarbonyl.

Bevorzugt verwendet werden erfindungsgemäße Verbindungen der allgemeinen Formel (I),
in welcher
- R¹: für Wasserstoff, Fluor, Chlor, C₁ -C₃-Alkyl oder C₁-C₃-Alkoxy steht,
- R²: für einen Rest der Formel 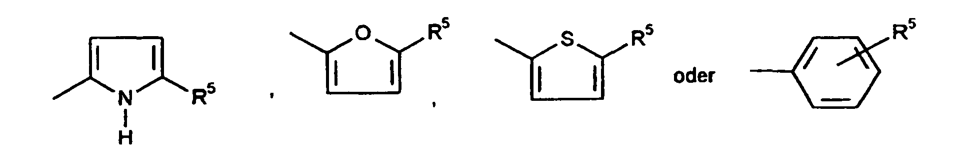 steht,
worin
R⁵ Wasserstoff, Chlor, Carboxyl, C₁-C₃-Alkyl, C₁-C₃-Alkoxycarbonyl oder einen Rest der Formel -CH₂-OR⁶ bedeutet,
worin
R⁶ Wasserstoff oder Methyl bedeutet,
- R³ und R⁴: gemeinsam einen Rest der Formel 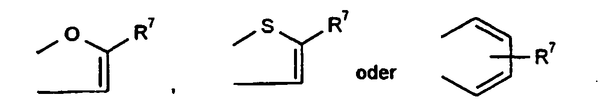 bilden,
worin
R⁷ Wasserstoff, Fluor, Chlor, C₁-C₃-Alkyl oder C₁-C₃-Alkoxy bedeutet,
und deren isomere Formen und Salze,
zur Behandlung von Erkrankungen, die mit Arzneimitteln mit vasodilatorischer Wirkung behandelbar sind.

Besonders bevorzugt verwendet werden erfindungsgemäße Verbindungen der allgemeinen Formel (I),
in welcher
- R¹: für Wasserstoff Fluor, Chlor oder Methoxy steht,
- R²: für einen Rest der Formel 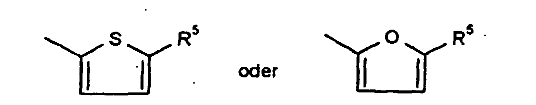 steht,
worin
R⁵ Wasserstoff, C₁-C₃-Alkyl oder einen Rest der Formel -CH₂-OR⁶ bedeutet,
worin
R⁶ Wasserstoff oder Methyl bedeutet,
- R³ und R⁴: gemeinsam einen Rest der Formel 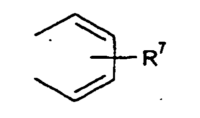 bilden,
worin
R⁷ Wasserstoff, Chlor, Fluor, Methyl oder Methoxy bedeutet,
und deren isomere Formen und Salze,
zur Behandlung von Erkrankungen, die mit Arzneimitteln mit vasodilatorischer Wirkung behandelbar sind.

Die Erfindung betrifft außerdem neue Stoffe, die in der folgenden Tabelle aufgeführt sind:

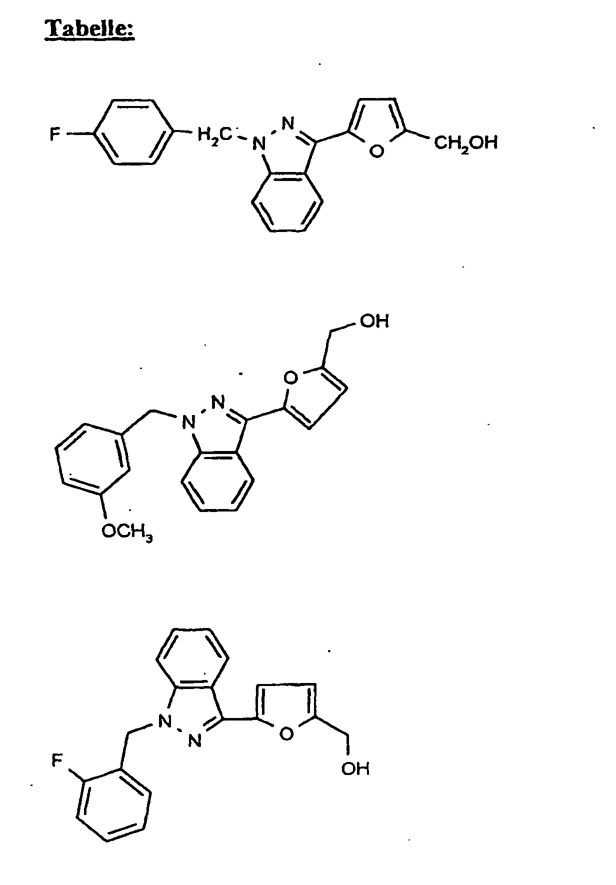

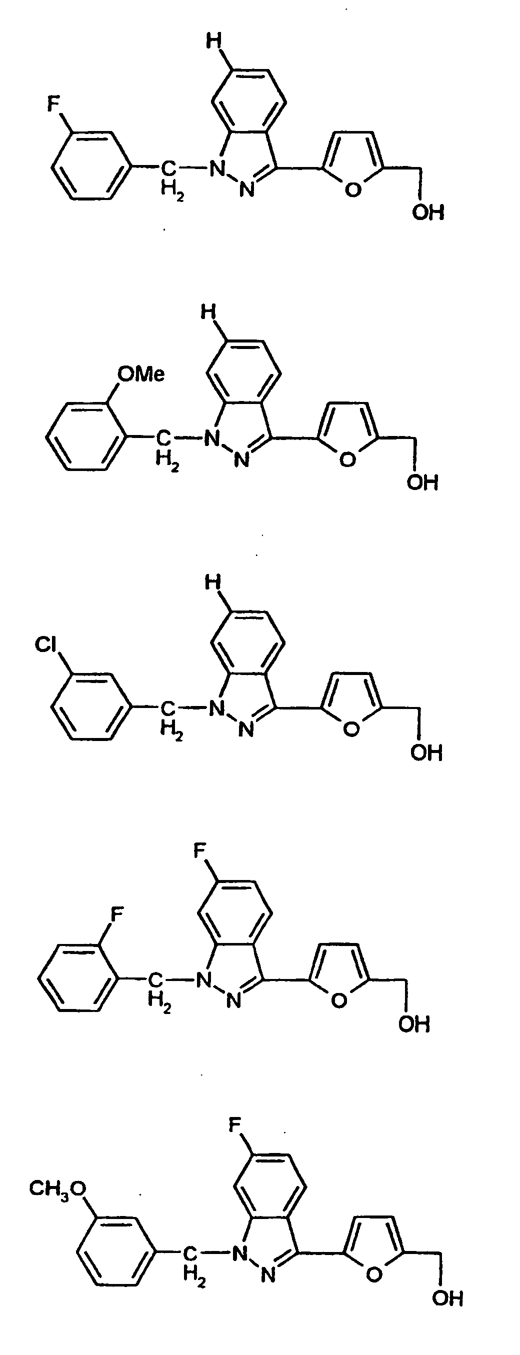

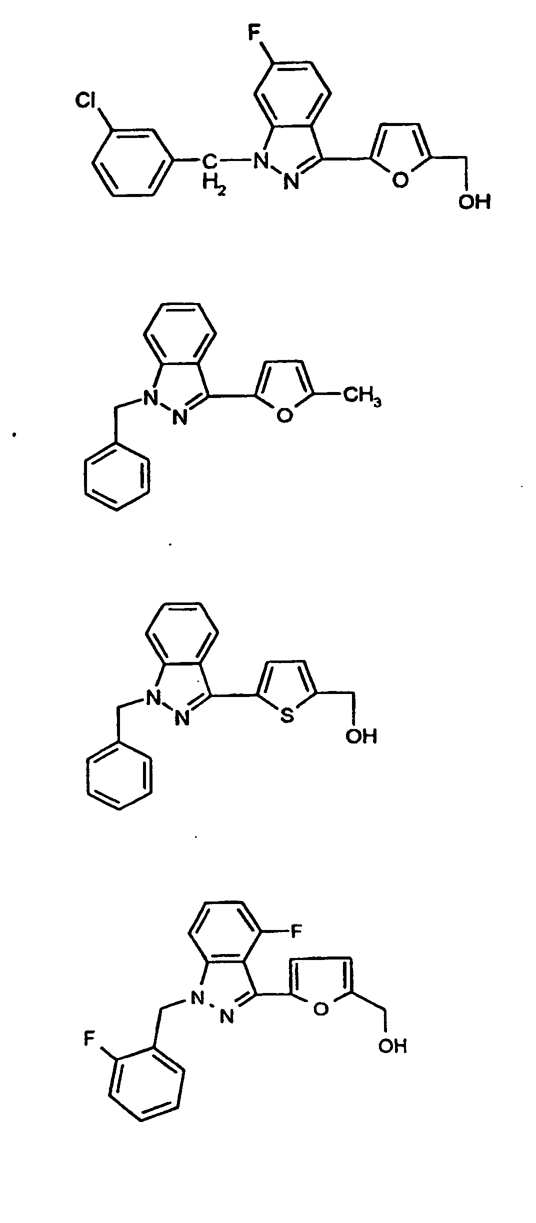

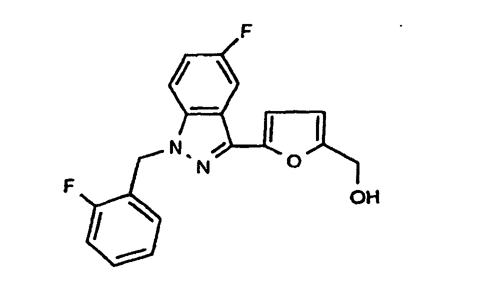

Die bekannten und neuen erfindungsgemäßen Verbindungen der allgemeinen Formel (I) können nach üblichen Methoden, z.B. gemäß EP-667 345 A1, hergestellt werden.

Darüber hinaus umfaßt die Erfindung vorzugsweise auch die Kombination der erfindungsgemäßen Verbindungen der allgemeinen Formel (I) und der neuen Stoffe mit organischen Nitraten und NO-Donoren.

Organische Nitrate und NO-Donoren im Rahmen der Erfindung sind im allgemeinen Substanzen, die über die Freisetzung von NO bzw. NO-Species ihre therapeutische Wirkung entfalten. Bevorzugt sind Natriumnitroprussid (SNP), Nitroglycerin, Isosorbiddinitrat, Isosorbidmononitrat, Molsidomin und SIN-1 und ähnliche Stoffe.

Außerdem umfaßt die Erfindung die Kombination mit Verbindungen, die den Abbau von cyclischem Guanosinmonophosphat (cGMP) inhibieren. Dies sind insbesondere Inhibitoren der Phosphordiesterasen 1, 2 und 5; Nomenklatur nach Beavo und Reifsnyder (1990) TIPS 11 S. 150-155. Durch diese Inhibitoren wird die Wirkung der erfindungsgemäßen Verbindungen potenziert und der gewünschte pharmakologische Effekt gesteigert.

Die erfindungsgemäß zu verwendenden neuen und bekannten Verbindungen der allgemeinen Formel (I) zeigen ein nicht vorhersehbares, wertvolles pharmakologisches Wirkspektrum. Sie induzieren z.B. eine Gefäßrelaxation und führen zu einer Blutdrucksenkung und Steigerung des koronaren Blutflusses.

Sie sind somit geeignet zur Verwendung bei der Behandlung von Erkrankungen, die mit Arzneimitteln mit vasodilatorischer Wirkung behandelbar sind wie beispielsweise der verschiedenen Formen der Angina pectoris, des Myokardinfarktes, der Herzinsuffizienz, der Arteriosklerose, Stroke und der Hypertonie.

Zur Feststellung der kardiovaskulären Wirkung wurden folgende Untersuchungen durchgeführt: In in vitro-Untersuchungen an Zellen vaskulären Usprungs wurde der Einfluß auf die Guanylatzyklase-abhängige cGMP-Bildung mit und ohne NO-Donor geprüft. Die gefäßrelaxierende Wirkung wurde an mit Phenylephrin vorkontrahierten Kaninchenaortenringen bestimmt. Die blutdrucksenkende Wirkung wurde an narkotisierten Ratten untersucht.

### Stimulation der löslichen Guanylatzyklase in primären Endothelzellen

Primäre Endothelzellen wurden aus Schweineaorten durch Behandlung mit Kollagenase-Lsg. isoliert. Anschließend wurden die Zellen in Kulturmedium bis zum Erreichen der Konfluenz kultiviert. Für die Untersuchungen wurden die Zellen passagiert, in Zellkulturplatten ausgesät und bis zum Erreichen der Konfluenz subkultiviert. Zur Stimulation der endothelialen Guanylatzyklase wurde das Kulturmedium abgesaugt und die Zellen einmal mit Ringerlösung gewaschen und in Stimulationspuffer mit oder ohne NO-Donor (Natrium-Nitroprussid, SNP, 1 µM) inkubiert. Im Anschluß daran wurden die Testsubstanzen (Endkonzentration 1 µM) zu den Zellen pipettiert. Nach Ende der 10-minütigen Inkubationszeit wurde die Pufferlösung abgesaugt und die Zellen 16 Stunden lang bei -20°C lysiert. Anschließend wurde das intrazelluläre cGMP radioimmunologisch bestimmt.

**Tabelle A**

| Bsp.-Nr. | % cGMP-Steigerung (NOSYNTH) |
|---|---|
| 1 | > 1000 |
| 2 | 72 |
| 3 | 250 |
| 4 | 413 |
| 7 | 734 |
| 8 | 28 |
| 10 | 238 |
| 11 | 14 |
| 14 (YC-1) EP 667 345 A1 | > 906 |

### Gefäßrelaxierende Wirkung in vitro

1,5 mm breite Ringe einer isolierten Kaninchen-Aorta werden einzeln unter einer Vorspannung in 5 ml-Organbäder mit 37°C warmer, carbogenbegaster Krebs-Henseleit-Lösung gebracht. Die Kontraktionskraft wird verstärkt und digitalisiert sowie parallel auf Linienschreiber registriert. Zur Erzeugung einer Kontraktion wird Phenylephrin dem Bad kumulativ in ansteigender Konzentration zugesetzt.

Nach mehreren Kontrollzyklen wird die zu untersuchende Substanz in jedem weiteren Durchgang in jeweils steigender Dosierung untersucht und mit der Höhe der im letzten Vordurchgang erreichten Kontraktion verglichen. Daraus wird die Konzentration errechnet, die erforderlich ist, um die Höhe des Kontrollwertes um 50 % zu reduzieren (IC₅₀). Das Standardapplikationsvolumen beträgt 5 µl.

**Tabelle B**

| Bsp.-Nr. | Aorta IC 50 (µM) |
|---|---|
| 1 | 4.1 |
| 3 | 16 |
| 4 | 9,2 |
| 14 (YC-1) EP 667 345 A | 10 |

### Blutdruckmessungen an narkotisierten Ratten

Männliche Wistar-Ratten mit einem Körpergewicht von 300 - 350 g werden mit Thiopental (100 mg/kg i.p.) anästhesiert. Nach Tracheotomie wird in die Femoralarterie ein Katheter zur Blutdruckmessung eingeführt. Die zu prüfenden Substanzen werden als Suspension in Tyloselösung mittels Schlundsonde in verschiedenen Dosen oral verabreicht.

**Tabelle C**

| Bsp.-Nr. | Dosis | max. Blutdrucksenkung | Zeit |
|---|---|---|---|
| 1 | 10 mg/kg | -14 mm Hg | 60 min |
| | 30 mg/kg | -18 mm Hg | 60 min |
| 14 (YC-1) | 10 mg/kg | -10 mm Hg | 60 min |
| EP 667 345 A1 | 30 mg/kg | -18 mm Hg | 60 min |

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchünittelaufnahme.

Weiterhin eignen sich diese Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Him-Traumas. Ebenso können die erfindungsgemäßen Verbindungen zur Bekämpfung von Schmerzzuständen eingesetzt werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

### Herstellungsbeispiele

### Beispiel 1

1-(2-Fluorbenryl)-3-(5-hydroxymethylfuran-2-yl)-indazol

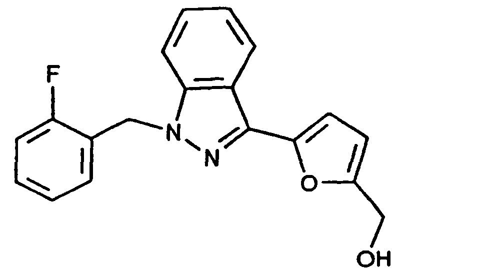

Man suspendiert 0.8 g (2.5 mmol) 1-(2-Fluorbenzyl)-3-(5-formyl-2-furanyl)-indazol in 40 ml Propanol und gibt bei 0°C langsam 0.8 g NaBH4 hinzu. Nach 1 Stunde Rühren bei Raumtemperatur gibt man die klare Lösung in Wasser, extrahiert mit Essigester, trocknet die organische Phase mit Natriumsulfat, verdampft im Vakuum und chromatographiert den Rückstand auf Kieselgel mit Toluol(T)/Essigester(E)-Gemischen als Eluens.

Man erhält 620 mg (77% d. Th.) Kristalle.
Smp. (Schmelzpunkt): 83°C
R_{f} (SiO₂, Toluol/Essigester 2:1): 0.50

Analog wurden die Beispiele in den Tabellen 1, 2 und 3 hergestellt:

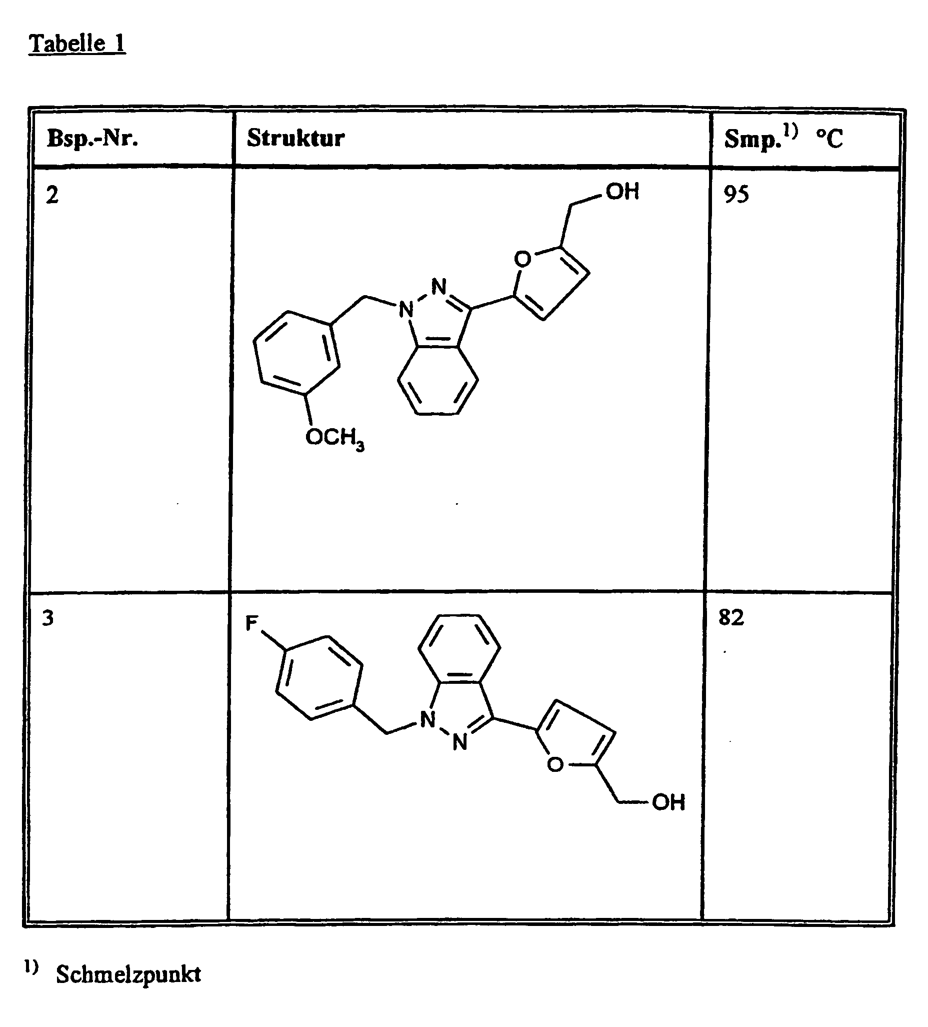

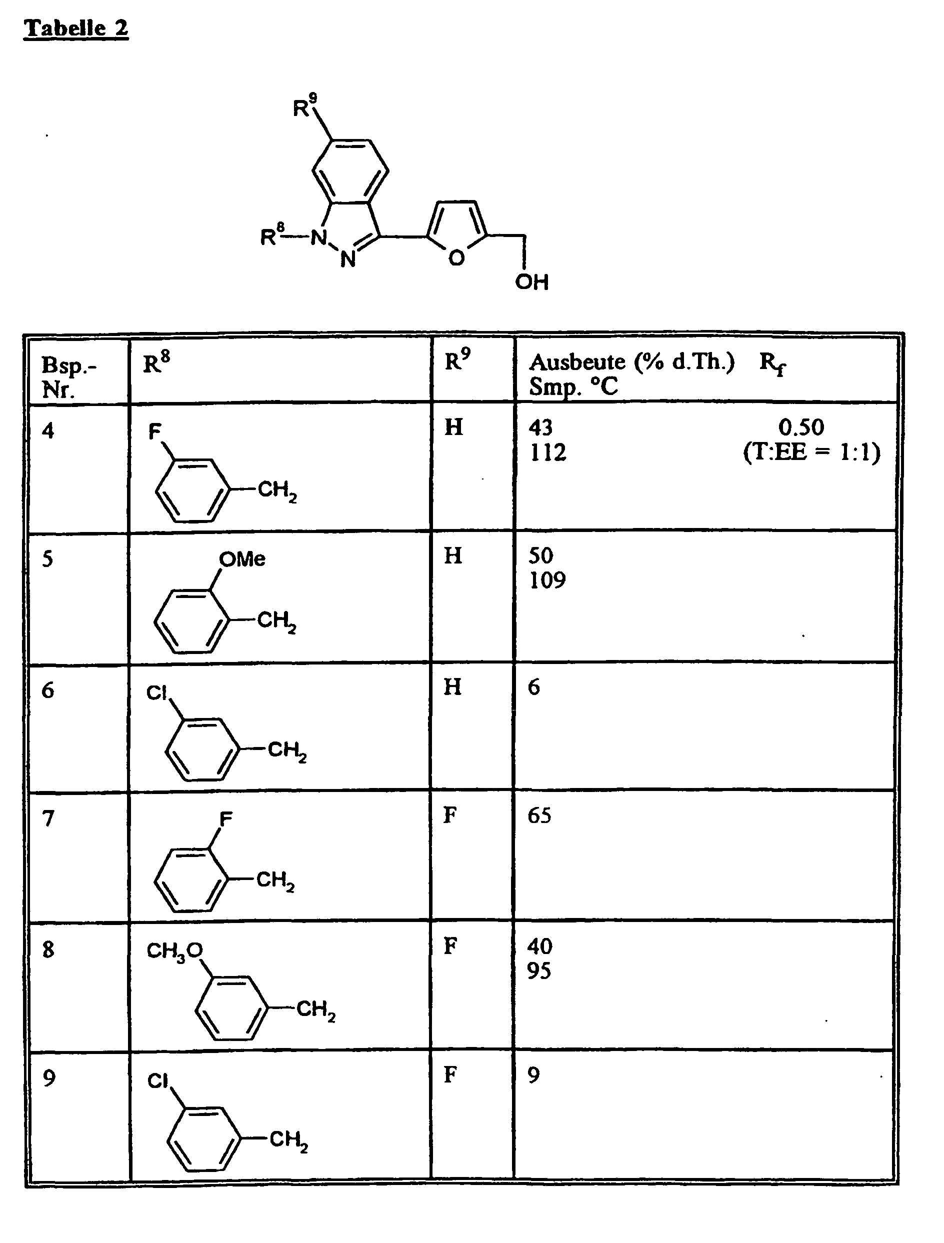

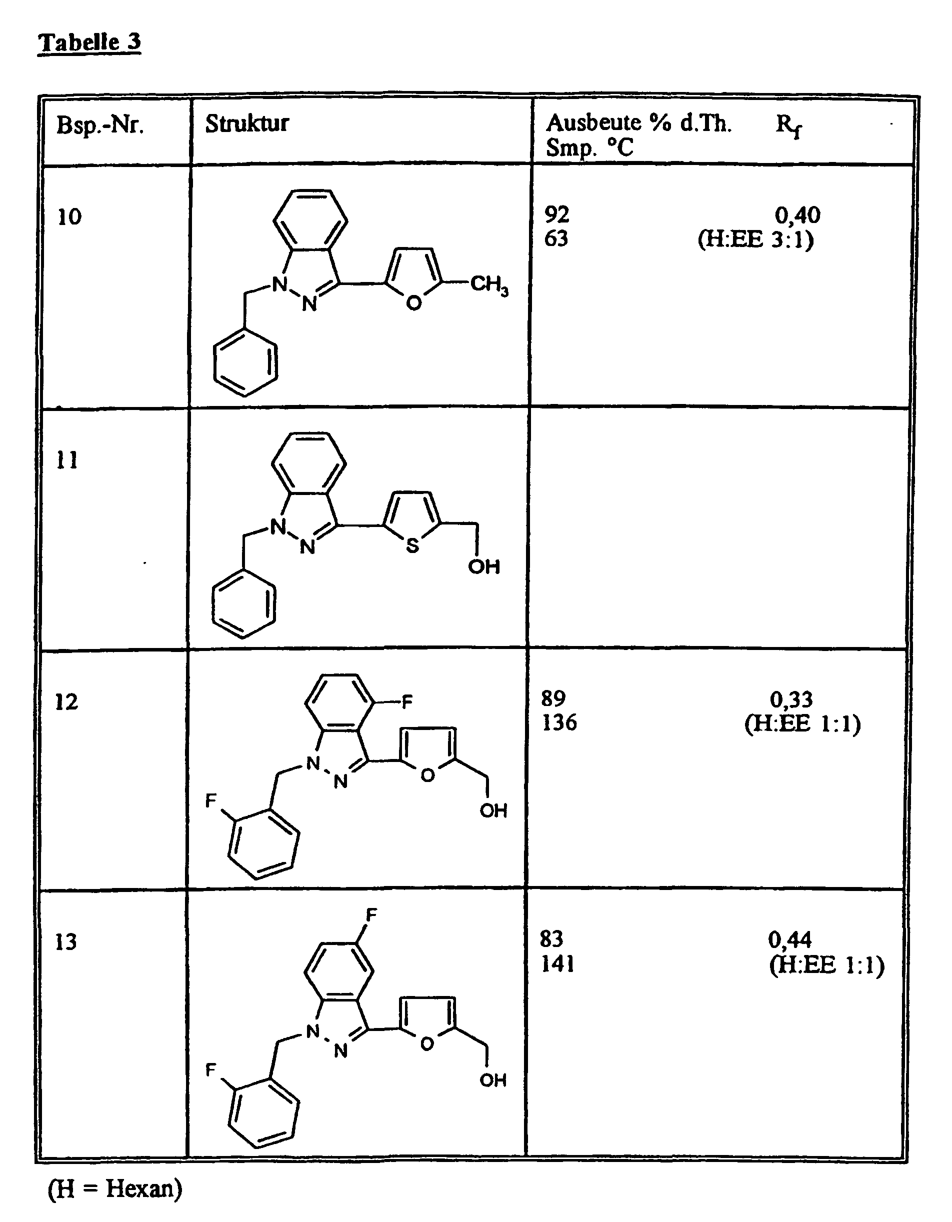

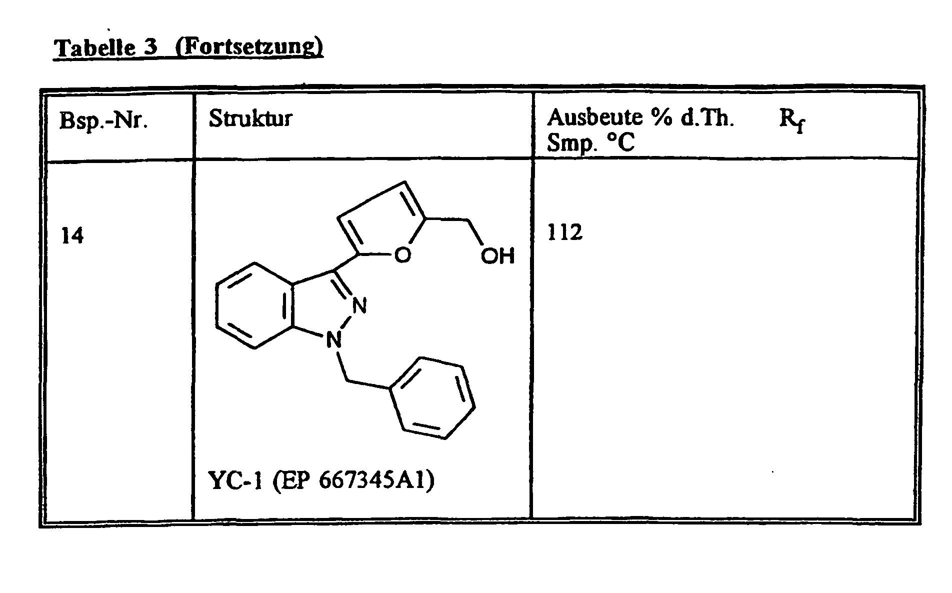

## Patentansprüche

1. Verwendung von 1-Benzyl-3-(substituierten hetaryl)-kondensietten Pyrazol-derivaten der allgemeinen Formel (I) 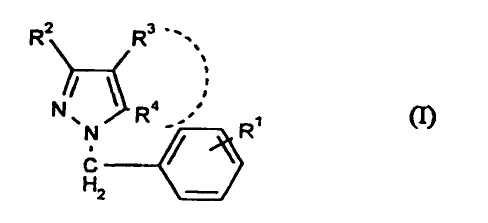 in welcher
R¹ für Wasserstoff, Halogen, Hydroxy oder C₁-C₃-Alkyl oder C₁-C₃-Alkoxy steht,
R² für einen Rest der Formel 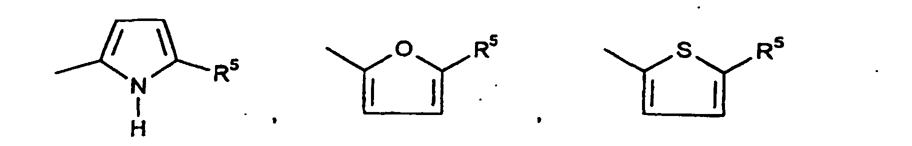 oder 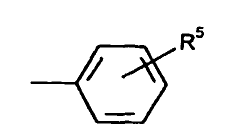 steht,
worin
R⁵ Wasserstoff, Halogen, Carboxyl, C₁-C₃-Alkyl, C₁-C₃-Alkoxycarbonyl oder einen Rest der Formel -CH₂-OR⁶ bedeutet,
worin
R⁶ Wasserstoff oder C₁-C₃-Alkyl bedeutet,
R³ und R⁴ gemeinsam einen Rest der Formel 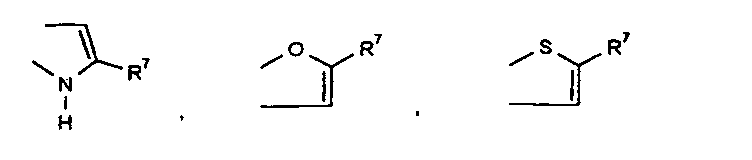 oder 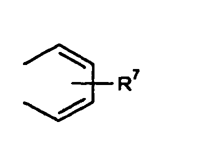 bilden,
worin
R⁷ Wasserstoff, Halogen, Hydroxy, C₁-C₃-Alkyl oder C₁-C₃-Alkoxy bedeutet,
und deren isomere Formen und Salze,
zur Herstellung von Arzneimitteln zur Behandlung von Erkrankungen die durch vasodilatorische Wirkung behandelbar sind.

2. Verwendung der Verbindungen der allgemeinen Formel (I) gemäß Anspruch 1 zur Herstellung von Arzneimitteln zur Bekämpfung von Angina pectoris, Myokardinfarzt, Herzinsuffizienz, Arteriosklerose, Stroke oder Hypertonie.

3. Verwendung gemäß Anspruch 2 zur Behandlung der Hypertonie.

4. Arzneimittel enthaltend Verbindungen der allgemeinen Formel (I) gemäß Anspruch 1 in Kombination mit organischen Nitraten und NO-Donoren.

5. Verwendung von Verbindungen der allgemeinen Formel (I) gemäß Anspruch 1 in Kombination mit organischen Nitraten und NO-Donoren zur Herstellung von Arzneimitteln zur Behandlung von Herz-Kreislauferkrankungen.

6. Verwendung von Verbindungen der allgemeinen Formel (I) gemäß Anspruch 1 in Kombination mit Verbindungen, die den Abbau von cGMP inhibieren, zur Herstellung von Arzneimitteln zur Behandlung von Herz-Kreislauferkrankunngen.

7. Neue Verbindungen aus der Gruppe
1-(2-Fluorbenryl)-3-(5-hydroxymethylfuran-2-yl)-indazol,
1-(4-Fluorbenzyl)-3-(5-hydroxymethylfuran-2-yl)-indazol,
3-(5-Hydroxymethylfuran-2-yl)-1-(3-methoxybenzyl)-indazol,
1-(3-Fluorbenzyl)-3-(5-hydroxymethylfiuan-2-yt)-indazol,
3-(5-Hydroxymethylfuran-2-yl)-1-(2-methoxybenzyl)-indazol,
1-(3-Chlorbenzyl)-3-(5-hydroxymethylfuran-2-yl)-indazol,
6-Fluor-1-(2-Fluorbenzyl)-3-(5-hydroxymethylfuran-2-yl)indazol,
6-Fluor-3-(5-hydroxymethylfuran-2-yl)-1-(3-methoxybenzyl)-indazol,
1-(3-Chlorbenzyl)-6-Fluor-3-(5-hydroxymethylfuran-2-yl)-indazol,
1-Benzyl-3-(5-methylfuran-2 yl)-indazol,
1-Benzyl-3-(5-hydroxymethylthien-2-yl)-indazol,
4-Fluor-1-(2-fluorbenzyl)-3-(5-hydroxymethylfuran-2-yl)-indazol und
5-Fluor-1-(2-fluorbenzyl)-3-(5-hydroxymethylfuran-2-yl)-indazol.

8. Arzneimittel enthaltend eine Verbindung, wie in Anspruch 7 definiert.

9. Verwendung gemäß Anspruch 1 zur Behandlung von Erkrankungen des Zentralnervensystems.

10. Verwendung gemäß Anspruch 9 zur Behandlung von cerebralen Infarkten.

## Claims

1. Use of 1-benzyl-3-(substituted hetaryl)-fused pyrazole derivatives of the general formula (I) 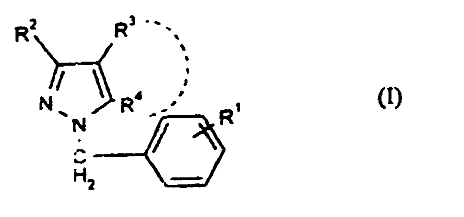 in which
R¹ represents hydrogen, halogen, hydroxyl or C₁-C₃-alkyl or C₁-C₃-alkoxy,
R² represents a radical of the formula 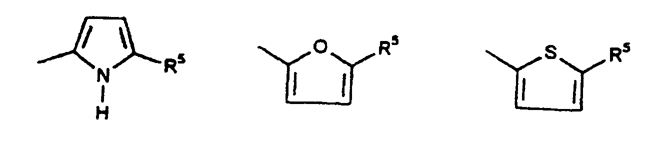 or 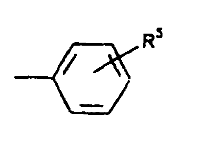 in which
R⁵ denotes hydrogen, halogen, carboxyl, C₁-C₃-alkyl, C₁-C₃-alkoxy-carbonyl or a radical of the formula -CH₂-OR⁶,
in which
R⁶ denotes hydrogen or C₁-C₃-alkyl,
R³ and R⁴ together form a radical of the formula 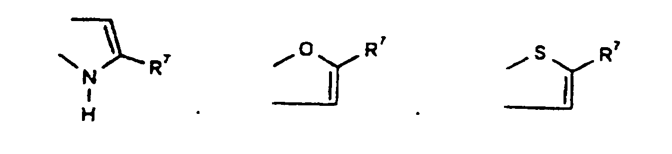 or 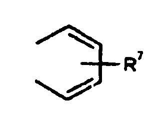 in which
R₇ denotes hydrogen, halogen, hydroxyl, C₁-C₃-alkyl or C₁-C₃-alkoxy,
and their isomeric forms and salts,
for the production of medicaments for the treatment of disorders which can be treated by vasodilatory action.

2. Use of the compounds of the general formula (I) according to Claim 1 for the production of medicaments for the control of angina pectoris, myocardial infarct, cardiac insufficiency, arteriosclerosis, stroke or hypertension.

3. Use according to Claim 2 for the treatment of hypertension.

4. Medicaments containing compounds of the general formula (I) according to Claim 1 in combination with organic nitrates and NO donors.

5. Use of compounds of the general formula (I) according to Claim 1 in combination with organic nitrates and NO donors for the production of medicaments for the treatment of cardiovascular disorders.

6. Use of compounds of the general formula (I) according to Claim 1 in combination with compounds which inhibit the degradation of cGMP, for the production of medicaments for the treatment of cardiovascular disorders.

7. Novel compounds from the group consisting of
1-(2-fluorobenzyl)-3-(5-hydroxymethylfuran-2-yl)-indazole,
1-(4-fluorobenzyl)-3-(5-hydroxymethylfuran-2-yl)-indazole,
3-(5-hydroxymethylfuran-2-yl)-1-(3-methoxybenzyl)-indazole,
1-(3-fluorobenzyl)-3-(5-hydroxymethylfuran-2-yl)-indazole,
3-(5-hydroxymethylfuran-2-yl)-1-(2-methoxybenzyl)-indazole,
1-(3-chlorobenzyl)-3-(5-hydroxymethylfuran-2-yl)-indazole,
6-fluoro-1-(2-fluorobenzyl)-3-(5-hydroxymethylfuran-2-yl)-indazole,
6-fluoro-3-(5-hydroxymethylfuran-2-yl)-1-(3-methoxybenzyl)-indazole,
1-(3-chlorobenzyl)-6-fluoro-3-(5-hydroxymethylfuran-2-yl)-indazole,
1-benzyl-3-(5-methylfuran-2-yl)-indazole,
1-benzyl-3-(5-hydroxymethylthiene-2-yl)-indazole,
4-fluoro-1-(2-fluorobenzyl)-3-(5-hydroxymethylfuran-2-yl)-indazole and
5-fluoro-1-(2-fluorobenzyl)-3-(5-hydroxymethylfuran-2-yl)-indazole.

8. Medicaments containing a compound as defined in Claim 7.

9. Use according to Claim 1, for the treatment of disorders of the central nervous system.

10. Use according to Claim 9 for the treatment of cerebral infarcts.

## Revendications

1. Utilisation de dérivés 1-benzyl-3-(hétaryle substitué)-pyrazoliques condensés de formule générale (I) 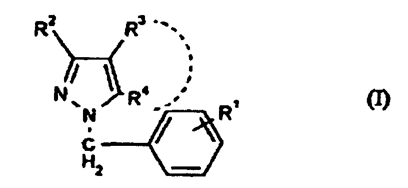 dans laquelle
R¹ représente l'hydrogène, un halogène, un groupe hydroxy ou un reste alkyle en C₁ à C₃ ou alkoxy en C₁ à C₃,
R² représente un reste de formule 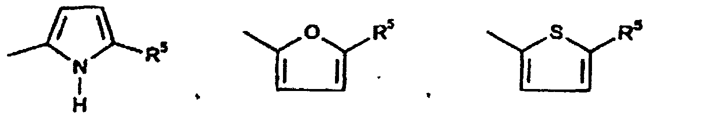 ou 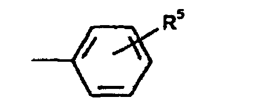 dans laquelle
R⁵ représente l'hydrogène, un halogène, un groupe carboxyle, un reste alkyle en C₁ à C₃, (alkoxy en C₁ à C₃) carbonyle ou un reste de formule -CH₂-OR⁶,
où
R⁶ représente l'hydrogène ou un reste alkyle en C₁ à C₃,
R³ et R⁴ forment ensemble un reste de formule 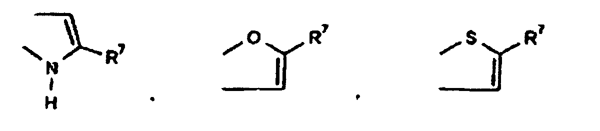 ou 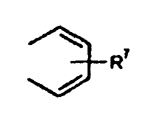 dans laquelle
R⁷ représente l'hydrogène, un halogène, un groupe hydroxy, un reste alkyle en C₁ à C₃ ou alkoxy en C₁ à C₃,
et leurs isomères et leurs sels,
pour la préparation de médicaments destinés au traitement de maladies qui peuvent être traitées par action vasodilatatrice.

2. Utilisation des composés de formule générale (I) suivant la revendication 1 pour la préparation de médicaments destinés à combattre l'angine de poitrine, l'infarctus du myocarde, l'insuffisance cardiaque, l'artériosclérose, l'attaque ischémique ou l'hypertonie.

3. Utilisation suivant la revendication 2, pour le traitement de l'hypertonie.

4. Médicament contenant des composés de formule générale (I) suivant la revendication 1 en combinaison avec des nitrates organiques et des donneurs de NO.

5. Utilisation de composés de formule générale (I) suivant la revendication 1 en combinaison avec des nitrates organiques et des donneurs de NO pour la préparation de médicaments destinés au traitement de maladies cardiovasculaires.

6. Utilisation de composés de formule générale (I) suivant la revendication 1 en combinaison avec des composés qui inhibent la dégradation du GMPc, pour la préparation de médicaments destinés au traitement de maladies cardiovasculaires.

7. Composés nouveaux du groupe
1-(2-fluorbenzyl)-3-(5-hydroxyméthylfurane-2-yl)-indazole,
1-(4-fluorbenzyl)-3-(5-hydroxyméthylfurane-2-yl)-indazole,
3-(5-hydroxyméthylfurane-2-yl)-1-(3-méthoxybenzyl)-indazole,
1-(3-fluorbenzyl)-3-(5-hydroxyméthylfurane-2-yl)-indazole,
3-(5-hydroxyméthylfurane-2-yl)-1-(2-méthoxybenzyl)-indazole,
1-(3-chlorbenzyl)-3-(5-hydroxyméthylfurane-2-yl)-indazole,
6-fluor-1-(2-fluorbenzyl)-3-(5-hydroxyméthylfurane-2-yl)-indazole,
6-fluor-3-(5-hydroxyméthylfurane-2-yl)-1-(3-méthoxybenzyl)-indazole,
1-(3-chlorbenzyl)-6-fluor-3-(5-hydroxyméthylfurane-2-yl)-indazole,
1-benzyl-3-(5-méthylfurane-2-yl)-indazole,
1-benzyl-3-(5-hydroxyméthylthiène-2-yl)-indazole,
4-fluor-1- (2-fluorbenzyl)-3-(5-hydroxyméthylfurane-2-yl)-indazole et
5-fluor-1-(2-fluorbenzyl)-3-(5-hydroxyméthylfurane-2-yl)-indazole.

8. Médicament contenant un composé tel que défini dans la revendication 7.

9. Utilisation suivant la revendication 1 pour le traitement de maladies du système nerveux central.

10. Utilisation suivant la revendication 9 pour le traitement d'infarctus cérébraux.
